# EUROPEAN PATENT SPECIFICATION

(11) **EP 0 401 271 B1**
(45) Date of publication and mention of the grant of the patent: **17.04.1996**
(21) Application number: 89902951.6
(22) Date of filing: 15.02.1989
(51) Int. Cl.: C07G 17/00, A61K 39/00, C12N 5/00, C12P 21/00

(54) **ANTIGEN AGAINST DIABETES**
ANTIGEN GEGEN DIABETES
ANTIGENE CONTRE LE DIABETE

(30) Priority: 18.02.1988 GB 8803756; 18.02.1988 GB 8803757
(43) Date of publication of application: 12.12.1990
(73) Proprietor: KS BIOMEDIX LTD., Esher, Surrey KT10 9RT (GB)
(72) Inventor: Tan, Kim Sze, Guildford, Surrey GU2 5PE (GB)
(74) Representative: Perry, Robert Edward
(86) International application number: GB8900138
(87) International publication number: WO8907601

(56) References cited:
- EP-A- 0 155 676
- EP-A- 0 251 107
- WO-A-87/05929
- GB-A- 2 146 338
- BIOCHEMICAL SOCIETY TRANSACTIONS, vol. 17, 1988; p. 519
- DIABETES, vol. 37, 1988; p. 206
- NATURE, vol. 298, 1982; pp. 167-169
- BIOCHEMICAL SOCIETY TRANSACTIONS, vol. 17, 1989; p. 650

## Description

### Field of the Invention

This invention relates to the use of a membrane antigen in the treatment of diabetes.

### Background of the Invention

Autoantibodies which are produced by B lymphocytes are involved in the pathogenesis of a number of autoimmune diseases, such as thyroiditis or diabetes, which involve organs such as the thyroid, pancreatic islets or adrenal glands. Some autoantibodies bind cell membrane receptors (or antigens) and trigger an immune response which leads to destruction of the cell or antigen. What triggers the autoimmune response is not known. Autoantibodies can also bind antigens, causing stimulation or blockage of biological processes. The circulating levels of autoantibodies in the body may be low, but such autoantibodies are highly potent and have to be neutralised to prevent damage to organs and cells.

Current evidence suggests that Type 1 diabetes mellitus is a chronic autoimmune disease involving two types of islet cell antibodies: (i) islet cell cytoplasmic antibodies (ICCA); (ii) islet cell surface antibodies (ICSA). The ICSA are believed to be central to the initial destruction of the beta cells, whereas ICCA is secondary to the damage. ICSA have been detected in 40-67% of recent-onset diabetics and are present in susceptible individuals long before clinical onset. As such, they may be used as an early predictor of diabetes and as a marker of the silent, ongoing beta cell damage in prediabetic individuals.

Various approaches have been adopted to neutralise autoantibodies, including the use of immunosuppressants, e.g. cyclosporin, and of mouse monoclonal anti-idiotypic antibody neutralisation techniques, but neither has been very successful. Immunosuppressants are too non-specific and will attack all B and T cells, while the use of mouse monoclonal anti-iodiotypic antibodies often results in the production of human anti-mouse antibodies in patients, considerably reducing the effectiveness of treatment.

Baekkeskov et al, Nature 298:167-169 (1982), identify a 64 K protein which immunoprecipitates with ICSA-positive sera.

As reported by Moyle et al, Biochem. Soc. Trans. 17:519 (1988), the search for the autoantigen recognised by ICSA-positive sera has been hampered by both the small amounts of antibody available and also its polyclonality. Moyle et al disclose a heterogenetic monoclonal anti-(antiiodiotypic) antibody obtained from a diabetic patient, that recognises a membrane glycoprotein. Moyle et al, Diabetes 37:206 (1988), also disclose an anti-anti-iodiotypic mouse monoclonal antibody.

### Summary of the Invention

According to the present invention, a purified pancreatic islet cell membrane antigen, or an epitope thereof, is used for the manufacture of a medicament for use in the prevention or treatment of diabetes.

### Detailed Description of the Invention

Epitopes containing the sequences specific for locking autoantibodies can be identified by standard gene-sequencing techniques. Once the amino-acid sequence is known, the peptide fragments may be synthetically produced. Synthetic production, e.g. by recombinant technology, may be the preferred route for epitopes that are composed of amino-acids only. However, for epitopes which are glycoproteins, lipoproteins or glycolipids, synthesis is less easy. The preferred route to produce such antigenic determinants is from hybrid cell lines.

Enzyme digestion or chemical treatment of antigens can be carried out to produce individual epitopes which are able to bind specifically to the autoantibodies, so causing a neutralising effect.

Epitopes of the invention are essentially free of naturally-associated material. They may be purified by standard techniques. Examples of such techniques are affinity purification, HPLC and electrophoresis.

A epitope of the invention may be incorporated into a composition which can be used to treat diabetic patients.

Long-term treatment of autoimmune diseases may involve not only the neutralisation but also the destruction of the B lymphocytes which secrete autoantibodies. Epitopes of the invention can be tagged with radioactive tracers such as iodine or cytotoxic drugs which can then be used to target and destroy autoantibody-secreting B lymphocytes.

Tagging may be of particular value in the prevention or prophylaxis of diabetes, where it is believed that antoantibodies can be detected 5 to 7 years before the patient becomes insulin-dependent; there may therefore be adequate time for individuals to be treated with neutralising agents such as epitopes specific for the autoantibody, up to the stage when 50% (or more) of the pancreas has been destroyed.

Epitopes may also be used to screen biological samples from individuals before an autoimmune disease is manifested. For screening, the epitopes may be incorporated into diagnostic kits.

By the procedure of the following illustrative Example, islet cell membrane antigens (ICMA) have been purified from cell lines that express the antigen and have demonstrated binding of the ICMA with human islet cell autoantibodies (ICA). The ICMA can be digested by a variety of enzymes or by chemical treatment, to give epitopes. ("Guildhay" refers to Guildhay Antisera Ltd., 6 Riverside Business Centre, Walnut Tree Close, Guildford, Surrey, England).

### Example

An anti-anti-idiotypic mouse monoclonal antibody (Moyle et al, 1988 Diabetes 37, 206; Moyle et al, 1988 Biochem. Soc. Trans., 17, 519) was used for the purification of the ICMA. 5 mg purified monoclonal antibodies were coupled on to 1 g activated beads (Guildhay). The column was washed 3 times with phosphate-buffered saline (PBS), and a solubilised islet cell membrane preparation, passed through a 0.2 µm filter (Millipore), was applied to the column. The column was then washed with PBS until there was no protein in the eluate. Bound ICMA was eluted at 4°C, and fractions containing ICMA were pooled and dialysed against PBS.

The purified ICMA was characterised in the following ways:
(a) Nitrocellulose blotting: Purified ICMA was spotted on to nitrocellulose membranes, incubated with ICSA-positive diabetic sera and anti-anti-idiotypic MAb. Staining was visualised by coomasie blue.
(b) Enzyme-linked immunosorbent assay (ELISA): Purified ICMA was coated on to sensitised microtitre wells, incubated with ICSA-positive diabetic sera and anti-anti-idiotypic MAb. Binding was visualised by adding either anti-human-HRP or anti-Mouse-HRP conjugate (Tan et al, Diabetes 1988, 37:204).
(c) Displacement ELISA: The purified ICMA will displace binding of ICSA-positive diabetic sera in the ICSA ELISA kit available from Guildhay.
(d) SDS-gel Electrophoresis: By classical SDS polyacrylamide gel electrophoresis, the ICMA appeared as a single band with a molecular weight of 60,000-65,000.
(e) Nature: The ICMA is apparently a glycoprotein with sialic acid terminal groups. Neuraminidase treatment, however, did not affect the binding of the ICMA to ICSA-positive diabetic sera.

Because it is believed that circulating ICSA initiates beta cell damage when bound to ICMA, it is possible to use complementary peptides such as epitopes or chemicals to bind the ICMA and thus prevent ICSA binding. In this way, the beta cells could effectively be prevented from damage. The design of these "protective" peptides or chemical compounds follows from knowledge of the amino-acid sequence of the antigenic site of ICMA.

## Claims

1. Use of purified pancreatic islet cell membrane antigen, or an epitope thereof, for the manufacture of a medicament for use in the prevention or treatment of diabetes.

## Patentansprüche

1. Verwendung eines gereinigten Pankreas-Inselzellmembran-Antigens, oder eines Epitops davon, zur Herstellung eines Medikaments zur Verwendung bei der Verhütung oder Behandlung von Diabetes.

## Revendications

1. Utilisation d'antigène purifié de membrane de cellules d'îlots pancréatiques, ou d'un site antigénique de celui-ci, pour la fabrication d'un médicament destiné à être utilisé dans la prévention ou le traitement du diabète.
